# EUROPEAN PATENT SPECIFICATION

(11) **EP 3 830 116 B1**
(45) Date of publication and mention of the grant of the patent: **19.02.2025**
(21) Application number: 19844801.1
(22) Date of filing: 05.08.2019
(51) Int. Cl.: C07K 14/745, G01N 33/68, G01N 33/86

(54) **TEST TO ASSESS LIVER DISEASE SEVERITY**
TEST ZUR BEURTEILUNG DES SCHWEREGRADES EINER LEBERERKRANKUNG
TEST D'ÉVALUATION DE LA GRAVITÉ D'UNE MALADIE HÉPATIQUE

(30) Priority: 03.08.2018 US 201862714161 P
(43) Date of publication of application: 09.06.2021
(73) Proprietor: University of Cincinnati, Cincinnati, OH 45221-0623 (US)
(72) Inventor: LEWIS, Clayton, Blue Ash, OH 45236 (US); BOGDANOV, Vladimir, Cincinnati, OH 45202 (US); VAN DREDEN, Patrick, 29410 Plouneour Menez (FR)
(74) Representative: Plasseraud IP
(86) International application number: PCT/US2019/045092
(87) International publication number: WO 2020/028898

(56) References cited:
- WO-A1-2017/210147
- WO-A2-2008/054822
- US-A1- 2014 046 683
- US-A1- 2014 141 986
- CAVERSACCIO NATHALIE ET AL: "Alternatively Spliced Tissue Factor Levels in the Plasma of Patients with Liver Fibrosis, Liver Cirrhosis, and Hepatocellular Carcinoma: A Single-Center Retrospective Study", BLOOD, AMERICAN SOCIETY OF HEMATOLOGY, US, vol. 130, 8 December 2017 (2017-12-08), pages 3701, XP086631637, ISSN: 0006-4971, DOI: 10.1182/BLOOD.V130.SUPPL_1.3701.3701
- LEWIS CLAYTON S ET AL: "A Novel Plasma Panel for the Assessment of Chronic Liver Disease Severity", BLOOD, AMERICAN SOCIETY OF HEMATOLOGY, US, vol. 132, 29 November 2018 (2018-11-29), pages 3784, XP086590620, ISSN: 0006-4971, DOI: 10.1182/BLOOD-2018-99-114906
- CALDWELL ET AL.: "Coagulation disorders and hemostasis in liver disease: Pathophysiology and critical assessment of current management", HEPATOLOGY, vol. 44, no. 4, 27 September 2006 (2006-09-27), pages 1039 - 1046, XP055684355
- MORANGE ET AL.: "Current knowledge on the genetics of incident venous thrombosi s", JOURNAL OF THROMBOSIS AND HAEMOSTASIS, vol. 11, 30 June 2013 (2013-06-30), pages 111 - 121, XP055684362

## Description

### TECHNICAL FIELD

The present invention relates to an assessment method for liver disease severity as defined in the claim. More specifically, the method as defined in the claim uses a blood or plasma-based panel to assess chronic liver disease severity.

### BACKGROUND OF THE INVENTION

Coagulopathy and inflammation-driven vascular derangements are common in chronic liver disease (CLD). Hepatocytes produce many clotting and fibrinolytic factors and in CLD, biosynthesis of these factors is altered leading to thrombotic complications such as portal vein thrombosis (PVT). PVT is a major cause of morbidity and mortality in CLD. While some progress has been made recently with regard to the approaches to treat PVT, the ability to predict the onset of PVT and/or response to PVT management is lacking. Portal vein flow velocity is a useful parameter but there are no adequate markers and/or scores to predict PVT in CLD. Preventive anticoagulation is risky in CLD, and it is desirable to be able to identify patients for whom the benefit of thromboprophylaxis outweighs the risk of bleeding. Because systemic aberrations in hemostatic, inflammatory, and vascular systems are frequently observed in CLD, a blood panel that captures them may not only be a viable alternative to CLD staging in a point-of-care setting, but also be particularly useful in predicting PVT onset and/or response to PVT management.

The Child-Pugh score (CP) is a widely used tool to assess prognosis in patients with chronic liver disease (CLD) and cirrhosis. The score considers five factors, three of which assess the synthetic function of the liver (i.e., total bilirubin level, serum albumin, and international normalized ratio, or INR) and two of which are based on clinical assessment (i.e., degree of ascites and degree of hepatic encephalopathy).

The CP score is calculated as follows: Factor 1 - Encephalopathy: None (1 point), Grade 1: Altered mood/confusion (2 points), Grade 2: Inappropriate behavior, impending stupor, somnolence (2 points), Grade 3: Markedly confused, stuporous but arousable (3 points), Grade 4: Comatose/unresponsive (3 points). Factor 2 - Ascites: Absent (1 point), Slight (2 points), Moderate (3 points). Factor 3 - Bilirubin: <2 mg/dL (1 point), 2-3 mg/dL (2 points), >3 mg/dL (3 points). Factor 4 - Albumin: >3.5 g/dL (1 point), 2.8-3.5 g/dL (2 points), <2.8 g/dL (3 points). Factor 5 - Prothrombin time/International normalized ratio (PT/INR): Less than 4 seconds above control/INR <1.7 (1 point), 4-6 seconds above control/INR 1.7-2.3 (2 points), More than 6 seconds above control/INR >2.3 (3 points). The points for each of the five factors are summated to attain a CP score.

For Child Pugh scores, Class A ("Child-Pugh-A") is assigned for Child-Pugh assessments with 5 to 6 points. This is considered the least severe level of liver disease. The one- to five-year survival rate is 95%. Class B ("Child-Pugh-B") is assigned for assessments with 7 to 9 points. This is considered moderately severe liver disease, with a one- to five-year survival rate of 75%. Class C ("Child-Pugh-C") is assigned for assessments with 10 to 15 points. This is considered the most severe level of liver disease. The one- to five-year survival rate is 50%.

Critics of the Child-Pugh score have noted its reliance on clinical assessment, which may result in inconsistency in scoring. In particular, the usefulness of PT/INR has been questioned. Hepatocytes produce a variety of clotting and fibrinolytic factors. In chronic liver disease, biosynthesis of these factors is often altered leading to hemostatic aberrations.

Document WO 2017/210147 discloses a method for the diagnosis of a liver disease comprising measuring the level of at least one biomarker which is a serum metabolite chosen from a panel of Bile Acids, Free Fatty Acids, Amino Acids and Carbohydrates.

### SUMMARY OF THE INVENTION

We have discovered a novel blood or plasma-based panel to assess CLD severity. This panel comprises clotting (co)factors, i.e. fVIII, fV, protein S, and protein C; a marker of fibrinolysis (D-dimer), and markers of endothelial activation, i.e. soluble P-selectin and alternatively spliced Tissue Factor (asTF). Of these seven proteins, asTF is a recently discovered molecule. asTF may contribute to prothrombotic states and endothelial dysfunction due to its ability to activate integrin subsets on endothelial cells. We devised a seven-component scoring system that included asTF. Our scoring system was able to predict CLD severity correlating to the Child-Pugh score (CP) with high accuracy. The present invention assesses CLD severity without the use of PT/INR and/or such subjective parameters as the extent of ascites and hepatic encephalopathy.

The present invention relates to a method for assessing the presence or severity of liver disease in a subject and treating the subject as defined in the claim. The method as defined in the claim involves obtaining a blood or plasma sample from a subject, analyzing the sample and obtaining data concerning the activities or levels of the following seven proteins: protein C, factor V, factor VIII, protein S, D-dimer, sP-selectin, and asTF. A score is assigned to each of the seven protein activities and/or levels as follows: a) a protein C activity >65% is assigned a score of 0, a protein C activity of 35-65% is assigned a score of 1, and a protein C activity <35% is assigned a score of 2; b) a factor V activity >80% is assigned a score of 0, a factor V activity of 55-80% is assigned a score of 1, and a factor V activity <55% is assigned a score of 2; c) a factor VIII activity <140% is assigned a score of 0, a factor VIII activity of 140-200% is assigned a score of 1, and a factor VIII activity >200% is assigned a score of 2; d) a protein S activity >50% is assigned a score of 0, and a protein S activity <50% is assigned a score of 2; e) a D-dimer level <0.5 ng/ml is assigned a score of 0, a D-dimer level of 0.5-1 ng/ml is assigned a score of 1, and a D-dimer level >1 ng/ml is assigned a score of 2; f) a sP-selectin level >100 pg/mL is assigned a score of 0, a sP-selectin level of 50-100 pg/mL is assigned a score of 1, and a sP-selectin level <50 pg/mL is assigned a score of 2; and g) an asTF level <0.3 pg/mL is assigned a score of 0, an asTF level of 0.3-0.5 pg/mL is assigned a score of 1, and an asTF level >0.5 pg/mL is assigned a score of 2. The assigned scores of the seven proteins are summated to obtain a cumulative assessment value. The method next determines the presence or severity of liver disease in the subject based on the cumulative assessment value as follows: a) a cumulative assessment value of 0-1 is normal; b) a cumulative assessment value of 2-3 is the least severe level of liver disease, comparable to Child-Pugh-A; c) a cumulative assessment value of 4-7 is a moderate level of liver disease, comparable to Child-Pugh-B; and d) a cumulative assessment value of 8 or greater is the most severe level of liver disease, comparable to Child-Pugh-C. A subject having been identified with liver disease is susceptible to be administered a treatment, a therapeutic agent, or both based on the identified severity of the liver disease.

Herein disclosed is a method for assessing the presence or severity of liver disease in a subject and treating the subject which involves: obtaining a blood or plasma sample from a subject; analyzing the sample and obtaining data concerning the level of alternatively spliced Tissue Factor (asTF); incorporating the asTF level as an element in assigning a severity of liver disease score; determining the presence or severity of liver disease in the subject based on the severity of liver disease score; and administering to a subject having liver disease a treatment, a therapeutic agent, or both based on the identified severity of the liver disease (This method is not part of the invention). In one aspect, the method disclosed herein (not part of the invention) further involves obtaining data from the blood or plasma sample concerning the activities and/or levels of the following proteins: protein C, factor V, factor VIII, protein S, D-dimer, sP-selectin; and incorporating the protein activities and/or levels as elements in assigning a severity of liver disease score. In another aspect , the method disclosed herein (not part of the invention) further involves obtaining data from the blood or plasma sample concerning the activities and/or levels of the following proteins: factor V, factor VIII, protein S, D-dimer, sP-selectin; and incorporating said protein activities and/or levels as elements in assigning a severity of liver disease score. In yet another aspect, the method disclosed herein (not part of the invention) further involves obtaining data from the blood or plasma sample concerning the activities and/or levels of the following proteins: protein C, factor V, protein S, D-dimer, sP-selectin; and incorporating said protein activities and/or levels as elements in assigning a severity of liver disease score. In still another aspect, the method disclosed herein (not part of the invention) further involves obtaining data from the blood or plasma sample concerning the activities and/or levels of the following proteins: protein C, factor V, factor VIII, D-dimer, sP-selectin; and incorporating said protein activities and/or levels as elements in assigning a severity of liver disease score.

An assessment tool to determine the severity of liver disease is disclosed herein (not part of the invention). The assessment tool disclosed herein (not part of the invention) comprises assigning a score to each of seven protein activities and/or levels as follows: a) a protein C activity >65% is assigned a score of 0, a protein C activity of 35-65% is assigned a score of 1, and a protein C activity <35% is assigned a score of 2; b) a factor V activity >80% is assigned a score of 0, a factor V activity of 55-80% is assigned a score of 1, and a factor V activity <55% is assigned a score of 2; c) a factor VIII activity <140% is assigned a score of 0, a factor VIII activity of 140-200% is assigned a score of 1, and a factor VIII activity >200% is assigned a score of 2; d) a protein S activity >50% is assigned a score of 0, and a protein S activity <50% is assigned a score of 2; e) a D-dimer level <0.5 ng/ml is assigned a score of 0, a D-dimer level of 0.5-1 ng/ml is assigned a score of 1, and a D-dimer level >1 ng/ml is assigned a score of 2; f) a sP-selectin level >100 pg/mL is assigned a score of 0, a sP-selectin level of 50-100 pg/mL is assigned a score of 1, and a sP-selectin level <50 pg/mL is assigned a score of 2; and g) an asTF level <0.3 pg/mL is assigned a score of 0, an asTF level of 0.3-0.5 pg/mL is assigned a score of 1, and an asTF level >0.5 pg/mL is assigned a score of 2. The assigned scores of the seven proteins are summated to obtain a cumulative assessment value.

In another aspect of the disclosure (not part of the invention) , a severity of liver disease score based on the assessment tool is determined as follows: a) a cumulative assessment value of 0-1 is normal; b) a cumulative assessment value of 2-3 is the least severe level of liver disease, comparable to Child-Pugh-A; c) a cumulative assessment value of 4-7 is a moderate level of liver disease, comparable to Child-Pugh-B; and d) a cumulative assessment value of 8 or greater is the most severe level of liver disease, comparable to Child-Pugh-C.

### BRIEF DESCRIPTION OF THE DRAWINGS

The objects and advantages of the disclosed invention will be further appreciated in light of the following detailed descriptions and drawings in which:
FIG 1 is an illustration of the structures of the major pro-coagulant full-length TF (flTF) and asTF.
FIG 2 is a schematic of flTF's and asTF's modes of action on cell surfaces promoting angiogenesis, cell migration, and (cancer) cell proliferation.
FIG 3 is a graph showing asTF level in healthy test subjects and those with chronic liver disease.
FIG 4 is a graph showing asTF level in test subjects separated by diagnosed liver condition.
FIG 5 is a graph showing asTF level by Child-Pugh score.
FIG 6 is a graph showing how asTF levels in CLD plasma correlate with the levels of D-Dimer.
FIG 7 is a graph showing how asTF levels in CLD plasma correlate with the levels of CD63⁺ microvesicles (MVs).
FIG 8 is a graph showing how asTF levels in CLD plasma correlate with the levels of CD14⁺ MVs.
FIG 9 is graph showing how asTF levels in CLD plasma correlate with the levels of CD31⁺/CD41⁻ MVs.
FIG 10 is a graph showing the correlation of asTF levels in CLD plasma to levels of CD31⁺/CD41⁻ MVs with the addition of Child-Pugh scores.
FIG 11 is a series of western blots, whole cell lysates, demonstrating that cytokine stimulation results in protein increases.
FIG 12 is a graph showing relative TF activity vs. TNFα for 5×10³ whole cells. This graph shows that TF is active on TMNK1 cell surfaces.
FIG 13 is a western blot of TMNK1 cultured media. The western blot shows that both CD31 and asTF are secreted by stimulated TMNK1 cells.
FIG 14 is a graph showing MVs per mL in cultured media of TMNK1 cells non-stimulated vs stimulated with TNFα. The graph shows that TNFα promotes the release of MVs from cells into the media.

### DETAILED DESCRIPTION OF THE INVENTION

The details of one or more aspects of the presently-disclosed subject matter are set forth in this document. Modifications to aspects described in this document, and other aspects, will be evident to those of ordinary skill in the art after a study of the information provided herein.

While the following terms are believed to be well understood by one of ordinary skill in the art, definitions are set forth to facilitate explanation of the presently-disclosed subject matter. Unless defined otherwise, all technical and scientific terms used herein have the same meaning as commonly understood by one of ordinary skill in the art to which the presently-disclosed subject matter belongs.

Unless otherwise indicated, all numbers expressing quantities of ingredients, properties such as reaction conditions, and so forth used in the specification and claims are to "about." Accordingly, unless indicated to the contrary, the numerical parameters set forth in this specification and claims are approximations that can vary depending upon the desired properties sought to be obtained by the presently disclosed subject matter.

As used herein, the term "about," when referring to a value or to an amount of mass, weight, time, volume, pH, size, concentration or percentage is meant to encompass variations of in some aspects ±20%, in some aspects ±10%, in some aspectss ±5%, in some aspects ±1%, in some aspects ±0.5%, and in some aspects ±0.1% from the specified amount, as such variations are appropriate to perform the disclosed method.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "liver disease" means a disorder that causes the liver to malfunction or cease functioning all together.

The term "chronic liver disease" as used herein denotes a disorder of the liver that involves progressive destruction and regeneration of the liver parenchyma leading to fibrosis and cirrhosis. Chronic liver disease causes can be any condition that results in the gradual degradation and renewal of the tissue cells with a body's liver.

The term "comparable," as used herein, means of the same approximate value.

The terms "treat," "treatment," and "treating," as used herein, refer to a method of alleviating or abrogating a disease, disorder, and/or symptoms thereof in a subject.

As used herein, a "subject" refers to a mammal. Optionally, a subject is a human or non-human primate. Optionally, the subject is selected from the group consisting of mouse, rat, rabbit, monkey, pig, and human. In a specific aspect, the subject is a human.

One of the leading methods for diagnosing the severity of liver disease, Child-Pugh, involves clinical assessments and subjective parameters such as the extent of ascites and hepatic encephalopathy. We have discovered a method of diagnosing the severity of liver disease that is simpler and less subjective. For patients as well as physicians, non-subjective (e.g. all-labs based) scores are more reliable and "portable" between health care providers because they cannot be influenced by such subjective elements of physical examination as "degree of ascites" and/or mental status. This comprises a major advantage for the new system that we have discovered.

Also, our system is less prone to subjective adjustments by practitioners. For example, even though the CP scale is split into A, B, and C, there are sub-sections in each CP group that liver specialists often note and use for tracking individual patient's disease dynamics longer term. Since our new scoring system does not require clinical assessment beyond laboratory testing, we contend that it is more objective and reliable. In addition, our scoring system appears to be particularly reflective of the current / future hemostatic & vascular statuses of the patient. Its seven components are proteins participating in clot formation (or a result of clot breakdown: d-dimer) as well as markers of perturbed vasculature (asTF, soluble p-selectin).

We explored the patterns of plasma alterations relative to CP scores to establish a plasma-based panel that can help evaluate the extent of CLD severity without the use of PT/INR and/or such subjective parameters as the extent of ascites and hepatic encephalopathy.

Our novel panel comprises a combination of clotting (co)factors (fVIII, fV, protein S, and protein C); a marker of fibrinolysis (D-dimer), and markers of endothelial activation, i.e. soluble P-selectin and alternatively spliced Tissue Factor (asTF). We devised a seven-component scoring system that was able to predict CLD severity with a high correlation to previously assigned CP scores. In fact, when the seven parameters are used together, the method exhibited a 97.2% correlation to previously assigned CP scores. We also note that reducing the panel to six factors (by removing either protein C or factor VIII) still showed a correlation of over 95%.

Of the seven proteins in our panel, asTF is a recently discovered molecule. asTF may contribute to prothrombotic states and endothelial dysfunction due to its ability to activate integrin subsets on endothelial cells. We found that asTF activites track CLD severity, after studying asTF plasma levels in healthy subjects, patients suffering from stage F0-3 liver fibrosis, liver cirrhosis, as well as hepatocellular carcinoma (HCC). asTF activities were found to increase with worsening CP scores. In fact, we found that asTF activities are higher in subjects previously assigned a CP-C score vs those previously assigned a CP-B or A score.

### Treatment and Therapeutic agents (not part of the invention)

Once the severity of liver disease is determined using our method, the subject can receive treatment. The treatment will vary, based on the level of liver disease severity. For example, treatment for the least severe level of liver disease may include a weight loss program comprising, for example, a regimen of dieting and exercise. Treatment for the most severe level of liver disease may be a transplant.

Additionally, subjects may be administered one or more therapeutic agents for treating liver disease. Such therapeutic agents can include, for example, metformin (e.g., Glucophage XR, Bristol Myers Squibb, Princeton, N.J.), ursodiol, and ppar-gamma agonists (e.g., pioglitizaone).

An effective amount of a therapeutic agent may include a dosage sufficient to provide a medically desirable result in a subject having a particular disease or condition. The effective amount will vary with the particular disease or condition being treated, the age and physical condition of the subject being treated, the severity of the condition, the duration of the treatment, the nature of any concurrent therapy, the specific route of administration, and the like factors within the knowledge and expertise of the health practitioner. It will be recognized that when the therapeutic agent is used in acute circumstances, it can be used to prevent one or more medically undesirable results that typically flow from such adverse events. It is expected that dosages will range depending on the method of administration. In the event that a response in a subject is insufficient at the initial dosages applied, higher dosages (or effectively higher dosages by a different, more localized delivery route) may be employed to the extent that patient tolerance permits. Multiple dosages per day are contemplated to achieve appropriate systemic levels of the therapeutic agent.

### EXAMPLES

### Example 1

A study was conducted in CLD patients suffering from cirrhosis due to untreated hepatitis C virus (HCV) and/or excessive alcohol consumption and patients were assessed on a CP scale: CP-A, n=12; CP-B, n=19; CP-C, n=11. Healthy control subjects (n=30) were age & sex matched. The following was measured: pro-coagulant phospholipid, fXa, D-dimer, fVIII, sP-selectin, thrombomodulin, protein C, protein S, asTF, free TFPI, and TFPI/protein S ratio. The activities and/or levels of fVIII, D-dimer, and asTF increased with CLD severity while protein C, fV, protein S, and sP-selectin activities and/or levels decreased (Table 2). In combination, these seven parameters were able to predict CP scores with high accuracy. We devised a scoring system which assigns a numeric value (0, 1, or 2) for each parameter's range (Table 1). A cumulative score of <2 is normal, 2-3 CP-A, 4-7 CP-B, and ≥8 CP-C (Table 3). The system was 97.2% accurate at predicting the CP score: of the 72 plasma samples analyzed, there was 1 false positive (a healthy subject scored as a CP-A), and 1 CP-C scored as a CP-B. Omission of any of the seven components of the panel markedly lowered the scoring system's accuracy. Tables 1-3 are below:

**Table 1: Scoring System**

| **Assigned Score** | **protein C (%)** | **factor V (%)** | **factor VIII (%)** | **protein S (%)** | **D-dimer (ng/ml)** | **sP-selectin (pg/mL)** | **asTF (pg/mL)** |
|---|---|---|---|---|---|---|---|
| 0 | >65 | >80 | <140 | >50 | <0.5 | >100 | <0.3 |
| 1 | 35-65 | 55-80 | 140-200 | <50 | 0.5-1 | 50-100 | 0.3-0.5 |
| 2 | <35 | <55 | >200 | | >1 | <50 | >0.5 |

**Table 2: Study data**

| **Child-Pugh score** | **protein C (%)** | **factor V (%)** | **factor VIII (%)** | **protein S (%)** | **D-dimer (ng/ml)** | **sP-selectin (pg/mL)** | **asTF (pg/mL)** |
|---|---|---|---|---|---|---|---|
| Normal | 125.93 | 104.93 | 102.73 | 82.23 | 0.28 | 141.57 | 231.00 |
| CP-A | 58.58 | 91.00 | 114.17 | 54.58 | 0.66 | 152.42 | 321.67 |
| CP-B | 46.44 | 77.78 | 130.83 | 49.33 | 0.98 | 90.94 | 433.89 |
| CP-C | 35.64 | 58.18 | 163.18 | 49.27 | 2.05 | 58.73 | 508.18 |

**Table 3: Cumulative Assessment Score**

| | | | |
|---|---|---|---|
| NORMAL: 0-1 | CP-A: 2-3 | CP-B: 4-7 | CP-C: ≥8 |

Regarding Table 2, as CLD severity increased, activities and/or levels of fVIII, D-dimer and asTF increased while the activities and/or levels of protein C, fV, protein S, and sP-selectin decreased; the activities and/or levels of these seven proteins correlated to previously assigned CP scores with high accuracy. Our scoring system, which assigns a numeric value for each parameter (Table 1), exhibited 97.2% correlation to previously assigned CP scores. The correlation diminished if we removed any of the seven parameters. Table 4 shows the reduction in the degree of correlation to previously assigned CP scores when a given component is excluded from the panel.

**Table 4: CP Score Correlation**

| **Excluded Factor** | **protein C** | **factor V** | **factor VIII** | **Protein S** | **D-dimer** | **sP-selectin** | **asTF** |
|---|---|---|---|---|---|---|---|
| **% Accurate** | 95.77 | 88.73 | 95.77 | 94.36 | 90.14 | 91.56 | 90.14 |

As stated above, when the seven parameters are used together, the method exhibited a 97.2% correlation to previously assigned CP scores. Removing either the protein C or factor VIII parameter gave a 95.77 correlation. Removing the protein S parameter gave a 94.36 correlation.

### Alternatively spliced Tissue Factor

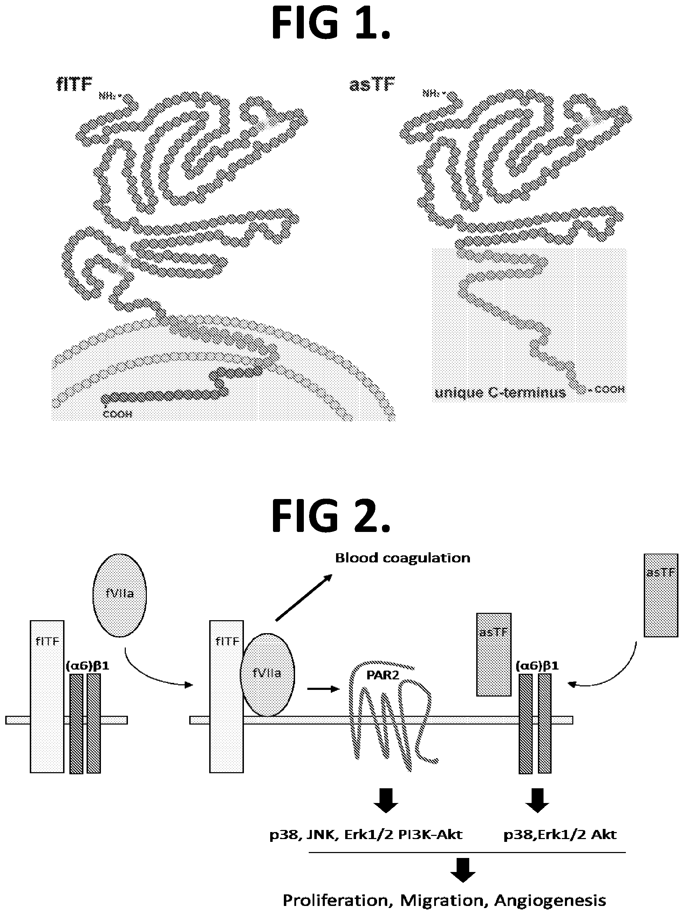

A novel element in this panel is alternatively spliced Tissue Factor (asTF), a soluble, secreted form of TF. Structures of the major pro-coagulant full-length TF (flTF) and asTF are shown in FIG. 1. FIG 2 is a schematic of flTF's and asTF's modes of action on cell surfaces promoting angiogenesis, cell migration, and (cancer) cell proliferation.

### Example 2

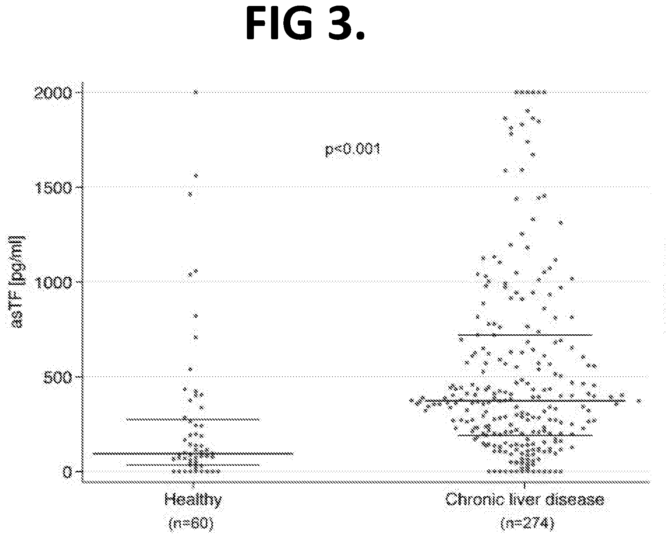

We analyzed asTF plasma levels in healthy subjects, patients suffering from stage F0-3 liver fibrosis, liver cirrhosis, as well as hepatocellular carcinoma (HCC). asTF plasma levels were measured using custom sandwich enzyme-linked immunosorbent assay (ELISA) prototypes. Values were expressed as median with interquartile range (IQR). The lowest median plasma asTF concentration (94 pg/ml, IQR: 33-275) was found in the healthy control group. Significant differences between asTF levels in the plasma of healthy subjects and those in grade F0-1 fibrosis patients (p<0.001), grade F2-3 fibrosis patients (p=0.019), cirrhosis patients (p=0.004), and HCC patients (p<0.001) were found using a Wilcoxon rank-sum test. asTF levels were found to increase with worsening CP scores (FIGs 3-5).

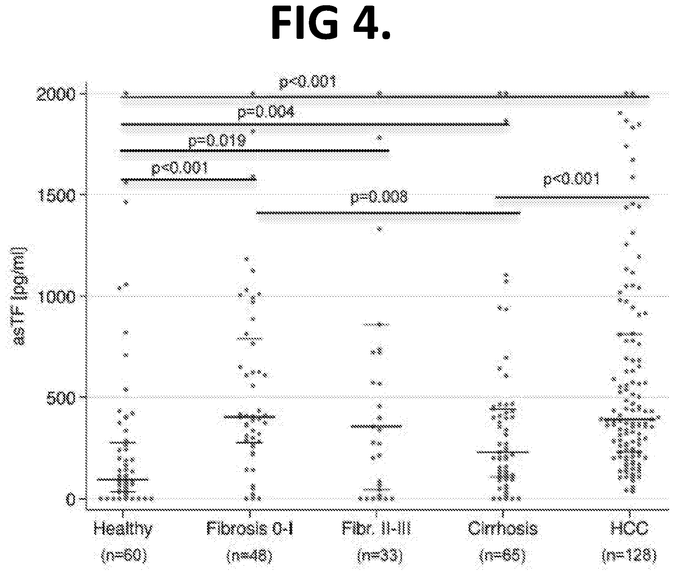

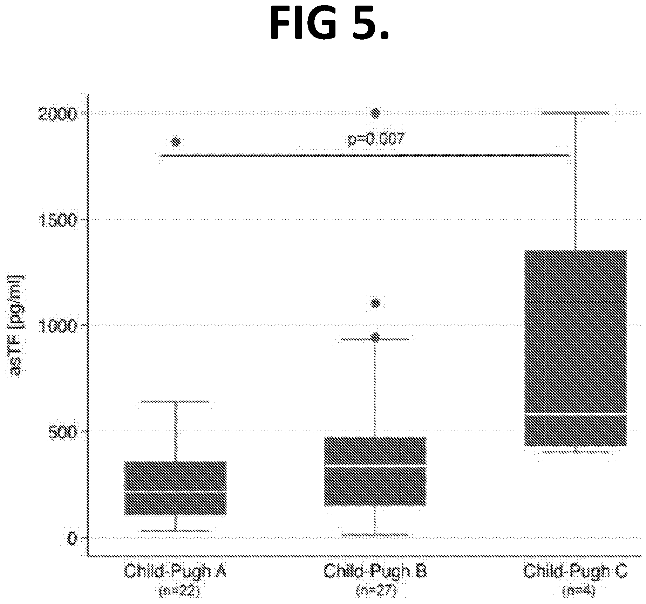

FIG 3 shows circulating asTF in healthy subjects (n=60) and patients with CLD (n=274). FIG 4 shows CLD sub-cohort breakdown as indicated. Thick bars, median asTF concentrations in plasma; thin bars, interquartile range. For illustration purposes, plasma asTF concentrations >2000 pg/ml are shown as 2000 pg/ml. FIG 5 shows circulating asTF levels in cirrhotic patients with CP-A (n=22), CP-B (n=27) and CP- C (n=4). asTF can be secreted by endothelial cells in response to pro-inflammatory cytokines; aberrant levels of coagulation factors as well as endothelial dysfunction are thought to contribute to CLD-associated coagulopathy. We found that asTF levels are higher in CP-C vs CP-B/A (FIG 5).

### Example 3

In another study, plasma was isolated from citrated whole blood. All CLD patients (n=42) were previously assigned a CP score; healthy controls (n=30) were age and sex matched. Consent was obtained. D-dimer, fV, fVIII, sP-selectin, protein C, protein S, and asTF were measured. Thrombin generation tests were performed. MVs of various origin were studied. Specified cutoffs for each parameter were derived using clinical ranges and the data generated in this study to compile a CLD severity score. Immortalized human liver sinusoidal endothelial cell line TMNK-1 was assessed for the expression of TF and MV release.

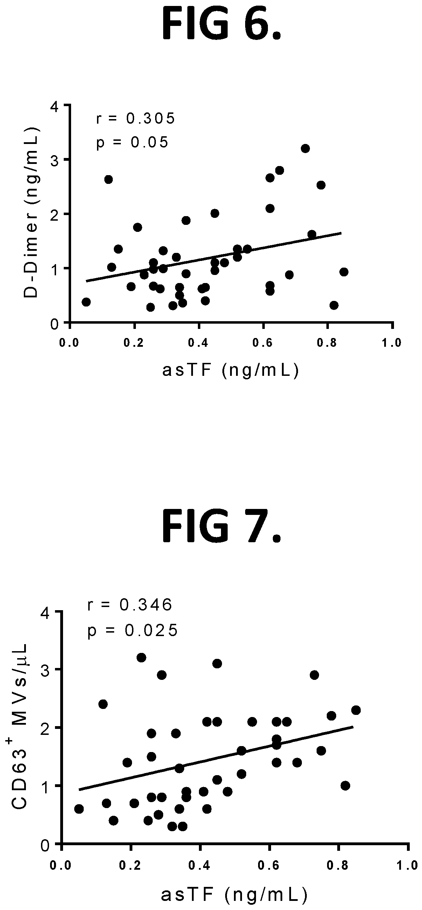

FIGs 6-10 concern patient plasma samples that were assayed for levels of asTF and D-dimer, CD63+ MVs, CD14+ MVs, and CD31+/CD41- MVs. asTF levels were found to correlate positively with D-dimer, CD63+ MVs and CD31+/CD41- MVs and negatively with CD14+ MVs. The strength of correlation between asTF and CD31+/CD41- MVs increased with CLD severity.

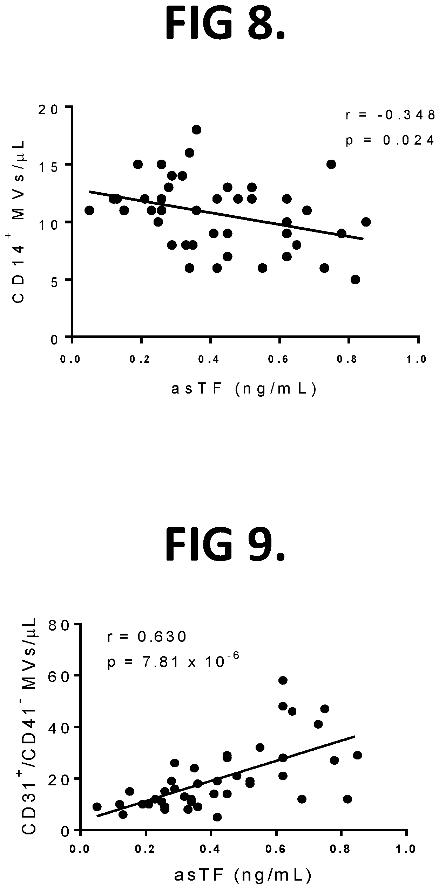

MVs are released in increased quantities by activated endothelial cells. To evaluate whether plasma asTF levels positively associate with the levels of endothelium-derived MVs, we measured the levels of CD31+/CD41- MVs in all CLD plasma samples by FACS. The levels of CD31+/CD41- MVs increased with disease severity (p<0.0001, data not shown). Strikingly, there was a strong and highly significant positive correlation between the levels of asTF and CD31+/CD41- MVs (r=0.63; p=7.81x10-6) (see FIG 9).

### Example 4

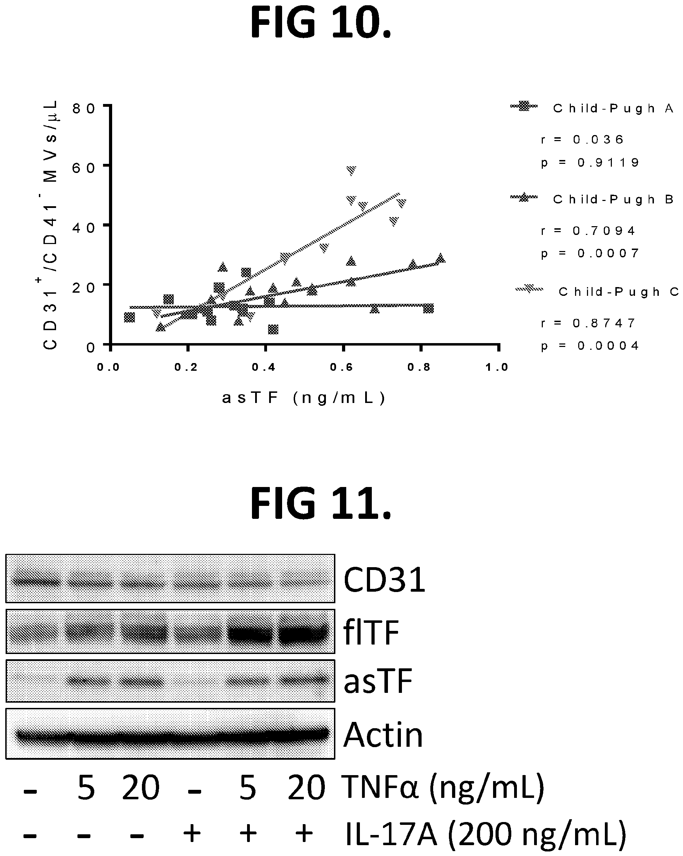

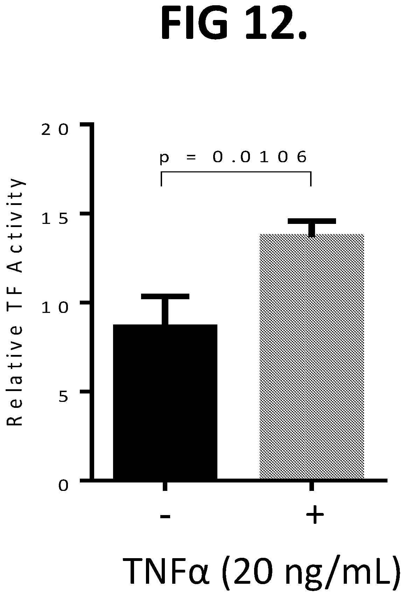

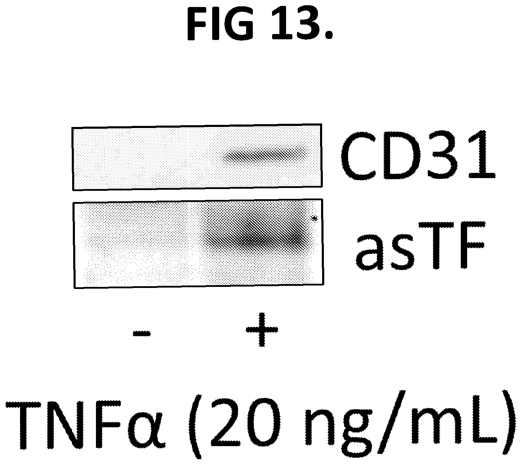

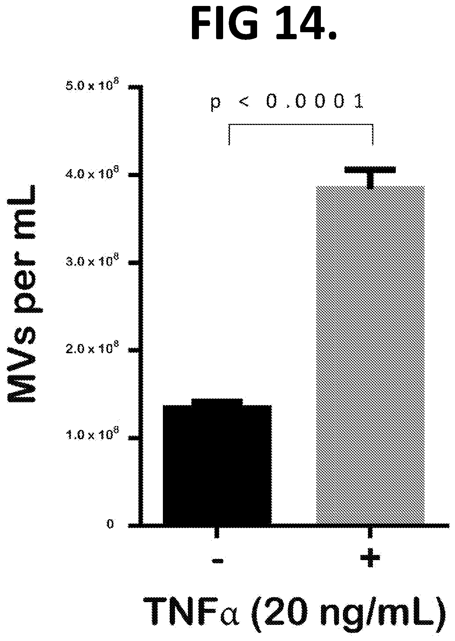

Given the strong positive correlation between plasma levels of asTF and CD31+/CD41-MVs in patients with CLD, we sought to determine whether cytokine stimulation of an immortalized human liver sinusoidal endothelium cell line (TMNK1) can mimic these observations in an *in vitro* setting. FIGs 11-14 show data regarding cytokine stimulation of TMNK1 cells and that such stimulation promotes TF expression, asTF secretion, and the release of MVs. TMNK1 cells were treated with TNFα -/+ IL-17A as indicated in each figure. FIG 11 shows western blots of whole cell TMNK1 lysates. The western blots demonstrate that cytokine stimulation results in protein increases. Actin was used as a loading control. FIG 12 is a TF enzymatic activity assay using 5×10³ intact cells. The assay demonstrated that TF is active on TMNK1 cell surfaces. In FIG 13, TMNK1 cells were placed in serum free medium and treated with TNFα for 24 hrs; medium was harvested, concentrated 30x and analyzed by western blot revealing that both CD31 (likely MV-bound) and asTF are secreted by stimulated TMNK1 cells. In FIG 14, TMNK1 cells were treated with TNFα for 24 hrs as indicated; media was collected and MVs concentrated by ultracentrifugation. MV levels were then determined using nanoparticle tracking analysis (NanoSight). TNFα stimulated the release of MVs from TMNK1.

In one aspect, the method of the present disclosure is a blood and/or plasma panel with utility in assessing CLD severity in a point-of-care setting. In another aspect, the method of the disclosure is useful in predicting PVT onset and/or response to PVT management. The scoring system of the present disclosure exhibits a 97.2% correlation to previously assigned CP scores and does not require clinical assessment beyond laboratory testing. In addition, we have shown that asTF levels positively correlate with those of CD31+/CD41- MVs and both increase with CLD severity.

The citation of any document is not to be construed as an admission that it is prior art with respect to the present invention.

It is to be further understood that where descriptions of various embodiments use the term "comprising," and / or "including" those skilled in the art would understand that in some specific instances, an embodiment can be alternatively described using language "consisting essentially of" or "consisting of."

## Claims

1. A method for assessing the presence or severity of liver disease in a subject, the method comprising:
a. analyzing a blood or plasma sample obtained from a subject and obtaining data concerning the activities or levels of the following seven proteins: protein C, factor V, factor VIII, protein S, D-dimer, sP-selectin, and asTF;
b. assigning a score to each of the seven protein activities or levels as follows:
i. a protein C activity >65% is assigned a score of 0, a protein C activity of 35-65% is assigned a score of 1, and a protein C activity <35% is assigned a score of 2;
ii. a factor V activity >80% is assigned a score of 0, a factor V activity of 55-80% is assigned a score of 1, and a factor V activity <55% is assigned a score of 2;
iii. a factor VIII activity <140% is assigned a score of 0, a factor VIII activity of 140-200% is assigned a score of 1, and a factor VIII activity >200% is assigned a score of 2;
iv. a protein S activity >50% is assigned a score of 0, and a protein S activity <50% is assigned a score of 2;
v. a D-dimer level <0.5 ng/ml is assigned a score of 0, a D-dimer level of 0.5-1 ng/ml is assigned a score of 1, and a D-dimer level >1 ng/ml is assigned a score of 2;
vi. a sP-selectin level >100 pg/mL is assigned a score of 0, a sP-selectin level of 50-100 pg/mL is assigned a score of 1, and a sP-selectin level <50 pg/mL is assigned a score of 2; and
vii. an asTF level <0.3 pg/mL is assigned a score of 0, an asTF level of 0.3-0.5 pg/mL is assigned a score of 1, and an asTF level >0.5 pg/mL is assigned a score of 2;
c. summating the assigned scores of the seven proteins to obtain a cumulative assessment value; and
d. determining the presence or severity of liver disease in the subject based on the cumulative assessment value as follows:
i. a cumulative assessment value of 0-1 is normal;
ii. a cumulative assessment value of 2-3 is the least severe level of liver disease, comparable to Child-Pugh-A;
iii. a cumulative assessment value of 4-7 is a moderate level of liver disease, comparable to Child-Pugh-B; and
iv. a cumulative assessment value of 8 or greater is the most severe level of liver disease, comparable to Child-Pugh-C.

## Patentansprüche

1. Verfahren zur Beurteilung des Vorliegens oder des Schweregrades einer Lebererkrankung bei einer Person, wobei das Verfahren Folgendes umfasst:
a. Analysieren einer Blut- oder Plasmaprobe, die von einer Person erhalten wurde, und Erhalten von Daten bezüglich der Aktivitäten oder Spiegel der folgenden sieben Proteine: Protein C, Faktor V, Faktor VIII, Protein S, D-Dimer, sP-Selektin und asTF;
b. Zuweisen einer Punktzahl zu jeder der sieben Proteinaktivitäten oder -spiegel wie folgt:
i. einer Protein C-Aktivität >65% wird eine Punktzahl von 0 zugewiesen, einer Protein C-Aktivität von 35-65% wird eine Punktzahl von 1 zugewiesen und einer Protein C-Aktivität <35% wird eine Punktzahl von 2 zugewiesen;
ii. einer Faktor-V-Aktivität >80% wird eine Punktzahl von 0 zugewiesen, einer Faktor-V-Aktivität von 55-80% wird eine Punktzahl von 1 zugewiesen und einer Faktor-V-Aktivität <55% wird eine Punktzahl von 2 zugewiesen;
iii. einer Faktor-VIII-Aktivität <140% wird eine Punktzahl von 0 zugewiesen, einer Faktor-VII I-Aktivität von 140-200% wird eine Punktzahl von 1 zugewiesen und einer Faktor-VIII-Aktivität >200% wird eine Punktzahl von 2 zugewiesen;
iv. einer Protein-S-Aktivität >50% wird eine Punktzahl von 0 zugewiesen und einer Protein-S-Aktivität <50% wird eine Punktzahl von 2 zugewiesen;
v. einem D-Dimer-Spiegel <0,5 ng/ml wird eine Punktzahl von 0 zugewiesen, einem D-Dimer-Spiegel von 0,5-1 ng/ml wird eine Punktzahl von 1 zugewiesen und einem D-Dimer-Spiegel >1 ng/ml wird eine Punktzahl von 2 zugewiesen;
vi. einem sP-Selektin-Spiegel >100 pg/ml wird eine Punktzahl von 0 zugewiesen, einem sP-Selektin-Spiegel von 50-100 pg/ml wird eine Punktzahl von 1 zugewiesen und einem sP-Selektin-Spiegel <50 pg/ml wird eine Punktzahl von 2 zugewiesen; und
vii. einem asTF-Spiegel <0,3 pg/ml wird eine Punktzahl von 0 zugewiesen, einem asTF-Spiegel von 0,3 - 0,5 pg/ml wird eine Punktzahl von 1 zugewiesen und einem asTF-Spiegel >0,5 pg/ml wird eine Punktzahl von 2 zugewiesen;
c. Summieren der zugewiesenen Punktzahlen der sieben Proteine, um einen kumulativen Beurteilungswert zu erhalten; und
d. Bestimmen des Vorliegens oder des Schweregrads einer Lebererkrankung bei der Person auf der Grundlage des kumulativen Beurteilungswertes wie folgt:
i. ein kumulativer Beurteilungswert von 0-1 ist normal;
ii. ein kumulativer Beurteilungswert von 2-3 ist der geringste Schweregrad einer Lebererkrankung, vergleichbar mit Child-Pugh-A;
iii. ein kumulativer Beurteilungswert von 4-7 ist ein mäßiger Grad einer Lebererkrankung, vergleichbar mit Child-Pugh-B; und
iv. ein kumulativer Beurteilungswert von 8 oder höher ist der schwerste Grad einer Lebererkrankung, vergleichbar mit Child-Pugh-C.

## Revendications

1. Procédé pour évaluer la présence ou la gravité d'une maladie hépatique chez un sujet, le procédé comprenant :
a. l'analyse d'un échantillon de sang ou de plasma obtenu à partir d'un sujet et l'obtention de données concernant les activités ou les niveaux des sept protéines suivantes : protéine C, facteur V, facteur VIII, protéine S, D-dimère, sP-sélectine et asTF ;
b. l'attribution d'un score à chacune des activités ou à chacun des niveaux des sept protéines comme suit :
i. une activité de protéine C > 65 % se voit attribuer un score de 0, une activité de protéine C de 35 à 65 % se voit attribuer un score de 1, et une activité de protéine C < 35 % se voit attribuer un score de 2 ;
ii. une activité de facteur V > 80 % se voit attribuer un score de 0, une activité de facteur V de 55 à 80 % se voit attribuer un score de 1, et une activité de facteur V < 55 % se voit attribuer un score de 2 ;
iii. une activité de facteur VIII < 140 % se voit attribuer un score de 0, une activité de facteur VIII de 140 à 200 % se voit attribuer un score de 1, et une activité de facteur VIII > 200 % se voit attribuer un score de 2 ;
iv. une activité de protéine S > 50 % se voit attribuer un score de 0, et une activité de protéine S < 50 % se voit attribuer un score de 2 ;
v. un niveau de D-dimère < 0,5 ng/ml se voit attribuer un score de 0, un niveau de D-dimère de 0,5 à 1 ng/ml se voit attribuer un score de 1, et un niveau de D-dimère > 1 ng/ml se voit attribuer un score de 2 ;
vi. un niveau de sP-sélectine > 100 pg/ml se voit attribuer un score de 0, un niveau de sP-sélectine de 50 à 100 pg/ml se voit attribuer un score de 1, et un niveau de sP-sélectine < 50 pg/ml se voit attribuer un score de 2 ; et
vii. un niveau de asTF < 0,3 pg/ml se voit attribuer un score de 0, un niveau de asTF de 0,3 à 0,5 pg/ml se voit attribuer un score de 1, et un niveau de asTF > 0,5 pg/ml se voit attribuer un score de 2 ;
c. le calcul de la somme des scores attribués des sept protéines pour obtenir une valeur d'évaluation cumulative ; et
d. la détermination de la présence ou la gravité d'une maladie hépatique chez le sujet sur la base de la valeur d'évaluation cumulative comme suit :
i. une valeur d'évaluation cumulative de 0 à 1 est normale ;
ii. une valeur d'évaluation cumulative de 2 à 3 est le niveau le moins grave de maladie hépatique, comparable à Child-Pugh-A ;
iii. une valeur d'évaluation cumulative de 4 à 7 est un niveau modéré de maladie hépatique, comparable à Child-Pugh-B ; et
iv. une valeur d'évaluation cumulative de 8 ou plus est le niveau le plus grave de maladie hépatique, comparable à Child-Pugh-C.
